# EUROPEAN PATENT SPECIFICATION

(11) **EP 2 908 860 B1**
(45) Date of publication and mention of the grant of the patent: **12.07.2023**
(21) Application number: 13784014.6
(22) Date of filing: 18.10.2013
(51) Int. Cl.: A61K 47/10, A61K 47/08, A61K 47/44, A61K 47/46, A61K 9/00, A61P 17/04

(54) **ENHANCED NAIL PENETRATING COMPOSITION**
VERBESSERTE NAGELDURCHDRINGUNGSZUSAMMENSETZUNG
COMPOSITION POUR LA PÉNÉTRATION DES ONGLES AMÉLIORÉE

(30) Priority: 19.10.2012 NL 2009669
(43) Date of publication of application: 26.08.2015
(73) Proprietor: Dutch Renewable Energy B.V., 1398 CP Muiden (NL)
(72) Inventor: PELLIKAAN, Hubert Clemens, NL-3572 CA Utrecht (NL)
(74) Representative: Patentwerk B.V.
(86) International application number: PCT/NL2013/050735
(87) International publication number: WO 2014/062059

(56) References cited:
- EP-A2- 1 138 314
- WO-A1-2009/048841
- WO-A2-2006/042324
- WO-A2-2011/079234
- US-A- 5 346 692
- US-A1- 2007 196 325

## Description

The present invention relates to a composition for use as a medicament wherein the composition is applied to a nail. The present invention further relates to a composition for use in the treatment of onychomycosis, and to a device comprising the composition for a medical use.

Onychomycosis is one of the most common medical conditions and it is an infection of the nail caused by fungi, e.g. dermatophytes and non-dermatophytes, and yeast, e.g. Candida species. Onychomycosis is classified in Candida onychomycosis, lateral and distal subungual, proximal subungual and superficial white. Toenails are in most cases affected by dermatophytes, e.g. Trichophyton Rubrum.

Although dermatophyte onychomycosis is relentlessly progressive there remains a view among some practitioners that it is a trivial cosmetic problem that does not merit treatment. However, in immunocompromised patients and the elderly the disease can give rise to complications such as paronychia and erysipelas, but also cellulitis which therefore further compromise the limb in those with diabetes or peripheral vascular disease. While these complications may not be common they are certainly serious. The high prevalence of the disease is the result of heavy contamination of communal bathing places by infected users; disinfecting the floors of such facilities is very difficult because fungal elements are protected in small pieces of keratin. It is therefore logical to try to reduce the number of infected users by effective treatment and thus reduce disease prevalence. Additionally, patients suffering from onychomycosis may experience nail pain or discomfort. Furthermore, onychomycosis is a surprisingly significant cause of medical consultation and of absence from work. In addition, also other type of nail diseases result in patients experiencing nail pain or discomfort.

Treatment of nail infections, including onychomycosis, is strenuous and nowadays both topical and oral medication is available for several nail diseases. For example, a permeating terbinafine formulation has been disclosed in WO 2011/079234 A2 wherein the composition comprises an anti-fungal agent, a zwitterionic surfactant or charged derivative thereof, a carboxylic acid, a lower alcohol and water.

A further example of topical medication is disclosed in EP 1 138 314 A2 describing a topical sustained release delivery system for delivery of antifungal agents to the finer or toenails achieving high penetration through the nails by combining the antifungal agent with a keratolytic agent and a humectant.

US 2007/0196325 A1 discloses solidifying adhesive formulations including an anti-infective drug, a solvent vehicle and a solidifying agent. Further US 5,346,692 describing a nail lacquer for treatment of onychomycosis wherein the nail lacquer comprises a film-forming agent, an antimycotically active substance and urea, wherewith the antimycotically active substance and the urea are liberated from the lacquer when the lacquer is applied.

Another example of a topically applied pharmaceutical composition is disclosed in WO 2006/042324 A2 wherein the composition typically includes an alkyl cellulose, a hydroxyalkyl cellulose, a pharmaceutically acceptable polar protic solvent, an antifungal agent selected from the group of naftifine, ciclopirox, terbinafine, pharmaceutically acceptable salts and combinations thereof, an glycol ether, an antipruritic agent selected from the group of camphor, menthol, butamben picrate, metacresol, benzyl alcohol, camphorated metacresol, juniper tar, phenol, phenolate sodium, resorcinol, camphorated metacresol, carbolic acid and combinations thereof, and a solubility enhancing agent, a surfactant, a wetting agent, or a combination thereof.

Particularly topical treatment of nail infections has several drawbacks in effectiveness and patient compliance. In order to improve effectiveness and patient compliance, the present invention provides a composition for delivering components topically to mammal nails, e.g. human fingernails and/or toenails, having enhanced nail penetrating properties. Penetration of the composition into the nail is essential for treatment of nail diseases, such as nail infections including onychomycosis. Therefore, due to the enhanced nail penetrating properties of the composition of the present invention the compounds present in the composition are absorbed by the nail in a more efficient way and therefore preventing the composition and its components to be wiped off after applying the composition onto the nail.

The present invention thereto provides a composition for use as a medicament wherein the composition is applied to a nail, according to appended claim 1. In particular the invention provides a composition for use in the treatment of onychomycosis, according to appended claim 2.

The composition of the present invention has improved nail penetration properties and therefore increases the effectiveness of the composition. It was found that the combination of ethanol as a water-miscible organic solvent, water as a hydrophilic solvent and a terpene and/or aetheric oil reduces the absorption time of the composition significantly. The absorption time of the composition of the present invention is less than 60 and even faster absorption times, e.g. less than 30 seconds, can be achieved as well. Such increase in nail penetration using the composition of the present invention provides a treatment that is more effective since the risk of removal of the composition before total absorption by the nail is reduced significantly. As a consequence, an increased amount of components applied to the nail is absorbed by the nail and therefore increases the effectiveness of the treatment. Additionally, the composition improves patient compliance, since the patient does not have to wait that long after applying the composition to the nail before the applied composition is completely absorbed by the nail. Furthermore, due to the enhanced penetration characteristics of the composition of the present invention, components present in the composition having volatile properties will be absorbed in higher amounts compared to compositions having less enhanced penetration characteristics. Therefore, by using the composition of the present invention, the person skilled in the art can better predict the amount of components that will be absorbed by the nail which leads to a more effective treatment of nail infections, such as onychomycosis.

It was found that in compositions comprising ethanol, water and a terpene and/or aetheric oil wherein the amount of water is more than 30% by weight of the total weight of the composition, droplets of water and terpene and/or aetheric oil were formed on the nail after applying the composition to the nail. These droplets are formed as a result of the evaporation of ethanol, due to the liquid/liquid phase split between ethanol and water. The nail did not absorb the droplets, which makes a composition having an amount of water of more than 30% by weight of the total weight of the composition ineffective for the treatment of nail infections, such as onychomycosis.

On the other hand compositions comprising ethanol and a terpene and/or aetheric oil, which is substantially free of water, a layer of terpene and/or aetheric oil remains on the nail after applying the composition to the nail. Such remainder will be wiped off by garments and therefore reducing the effectiveness of the composition.

Additionally, it was found that the penetration time was negatively influenced in compositions having a total amount of terpene and/or aetheric oil of more than 20% by weight of the total weight of the composition, partially due to the reduced solubility of the terpene and/or aetheric oil in those amounts in a mixture of water and a water-miscible solvent. Therefore in the composition according to the invention, the terpene and/or aetheric oil are present in a total amount of 1% to less than 15%. Preferably the terpene and/or aetheric oil are present in a total amount of about 1% to about 10% by weight of the total weight of the composition.

The water-miscible organic solvent used in the composition of the present invention is ethanol. Although, ethanol may evaporate after application of the composition to the nail, the majority of ethanol is absorbed into the nail due to the presence of water. According to the present invention, water is present in an amount of 10% to 25% by weight of the total weight of the composition. Preferably water is present in an amount of about 10% to about 20% by weight of the total weight of the composition. A composition comprising an amount of water in the range of about 10 wt-% to about 20 wt-% provides the most optimum absorption time. The actual absorption time of the composition of the present invention depends on which terpene and/or aetheric oil is used. Most preferably water is present in an amount of about 10%, about 15% or about 20% by weight of the total weight of the composition, depending on the terpene and/or aetheric oil used.

The term "about" as used herein is intended to include values, particular within 10% of the stated values.

The terpene and the aetheric oil used in the composition of the present invention can be selected from camphor, menthol, thymol, thyme oil, eucalyptus, eucalyptus citriodora, turpentine, pine oil, Melaleuca alternifolia, menthone, menthyl salicylate, musk, bixa orellana, borneol, turmeric oil, peppermint oil, clove oil, fennel oil, basil oil, patchouli oil, alpha pinene, terpineol, oregano oil, carvacrol and combinations thereof. The above-mentioned terpenes and aetheric oils are suitable for delivering components topically to mammal nails for use in the treatment of nail diseases, such as nail infections including onychomycosis. More preferably, compositions suitable for delivering components topically to mammal nails and having excellent nail penetration properties comprise terpenes and aetheric oils selected from thyme oil, thymol, eucalyptus citriodora, camphor, carvacrol and combinations thereof.

In even another embodiment of the present invention, the composition is formulated in a formulation, which is suitable for application to the nail. Therefore, the composition of the present invention can be in the form of a lotion, cream, ointment or gel.

The composition of the present invention is absorbed into the nail within 200 seconds, preferably within 120 seconds or even more preferably within 60 seconds. Preferably the composition of the present invention is absorbed into the nail within 45 seconds and most preferably the composition of the present invention is absorbed into the nail within 30 seconds.

According to the present invention the composition comprises lactic acid as an additive which is suitable for treatment of nail diseases, such as infections including onychomycosis. Lactic acid is used to lower the pH value of the nail and therefore preventing the fungi causing the infection to grow further. Optionally, urea may be used as a further additive to dissolve the structure of the nail and therefore can soften the nail, making it more porous and penetrable.

Preferably the composition according to the invention is applied to the nail in an amount of more than about 0,5 mg/cm², since lower amounts applied to the nail seems not to be effective in the treatment of nail diseases, such as onychomycosis. More preferably the composition is applied to the nail in an amount of more than about 2,0 mg/cm². Optimum results in effectiveness and nail absorption time are achieved wherein the composition is applied to the nail in an amount of about 4,5 mg/cm². Preferably the composition of the present invention is applied to the nail plate, however, the composition may also be applied to other parts of the nail, i.e. the lunula, nail groove and eponychium.

In even another aspect the present invention relates to a device comprising the composition for use as a medicament according to the first aspect of the invention, wherein the device is adapted to apply the composition to the to be treated area by dripping or smearing. Such device may further comprise metric means for the application of a measured quantity of the composition of the present invention to the nail. The device may be in the form of a brush, pen or flacon/dripping device.

The invention will now be further illustrated with reference to the following examples.

### Examples

Several compositions comprising ethanol, water and a terpene and/or aetheric oil were prepared by mixing the ingredients together in a beaker.

Additionally, the prepared compositions were tested by applying the compositions to nails of test persons using a brush. The time of complete absorption of the product was measured. When absorption was not complete and droplets were formed this was noted. An amount of 4.5 mg/cm2 was applied in each of these tests. The procedure was repeated several times. The nails of the test persons were cleaned and dried in between the tests. The results of the above absorption time tests are summarized in tables 1 and 2.

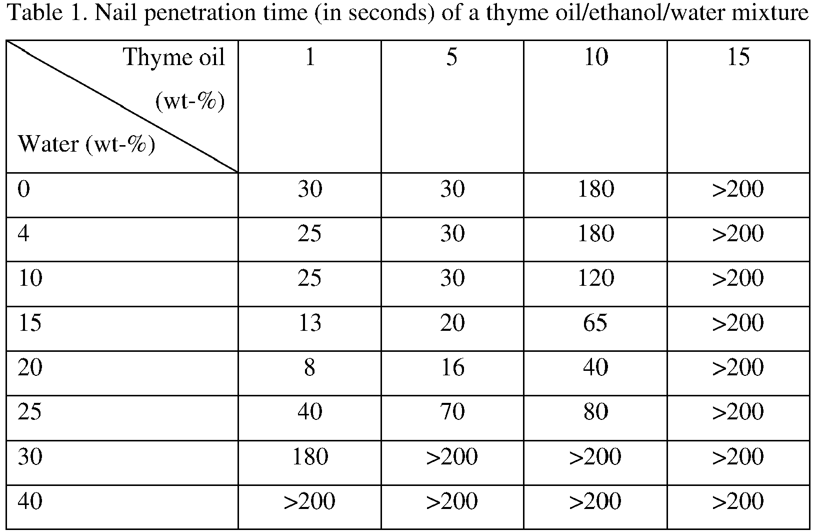

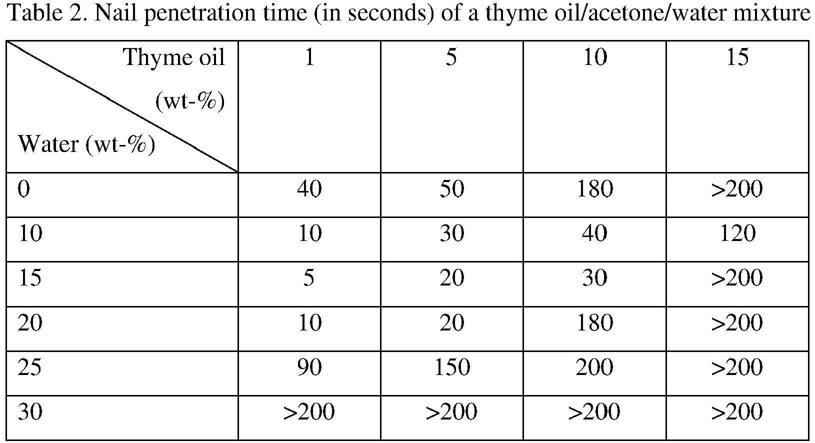

Additionally, several terpenes or aetheric oils were tested as well. Compositions comprising 10 wt-% terpenes or aetheric oils were mixed with different amounts of water and ethanol. The absorption time results are summarized in table 3.

**Table 3. Nail penetration time of 10 wt-% terpenes/aetheric oils in ethanol/water mixture**

| Terpene/Aetheric oil | Water (wt-%) | Penetration time (s) |
|---|---|---|
| Thyme oil | 20 | 40 |
| Thymol | 14 | 60 |
| Eucalyptus citriodora | 9 | 50 |
| Camphor | 16 | 25 |
| Carvacrol | 26 | 50 |

A composition for the treatment of onychomycosis was prepared according to the method described above. Table 4 provides the composition and the penetration time of the composition.

**Table 4. Nail penetration time composition for the treatment of onychomycosis**

| Component | Wt-% |
|---|---|
| Purified water | 17,3 |
| Ethanol (96%) | 68,1 |
| Lactic acid | 3,6 |
| Thyme oil (white) | 10,0 |
| Eucalyptus citriodora | 1,0 |
| Penetration time (s) | 43 |

## Claims

1. Composition for use as a medicament wherein the composition is applied to a nail, and the composition consists of:
- a water-miscible organic solvent which is ethanol;
- water;
- a terpene and/or aetheric oil
- an additive suitable for treatment of nail diseases which is lactic acid,
- and optionally urea as a further additive,
wherein:
- the water-miscible organic solvent is present in an amount of more than 50% by weight of the total weight of the composition;
- water is present in an amount of 10% to 25% by weight of the total weight of the composition; and
- the total amount of terpene and aetheric oil is 1% to less than 15% by weight of the total weight of the composition.

2. Composition for use in the treatment of onychomycosis, wherein the composition is applied to a nail, and the composition consists of:
- a water-miscible organic solvent which is ethanol;
- water;
- a terpene and/or aetheric oil,
- an additive suitable for treatment of nail diseases which is lactic acid,
- and optionally urea as a further additive,
wherein:
- the water-miscible organic solvent is present in an amount of more than 50% by weight of the total weight of the composition;
- water is present in an amount of 10% to 25% by weight of the total weight of the composition; and
- the total amount of terpene and aetheric oil is 1% to less than 15% by weight of the total weight of the composition.

3. Composition for a medical use according to any of the preceding claims, **characterized in that** water is present in an amount of 10% to 20% by weight of the total weight of the composition, most preferably water is present in an amount of 10%, 15% or 20% by weight of the total weight of the composition.

4. Composition for a medical use according to any of the preceding claims, **characterized in that** the terpene and the aetheric oil are selected from thyme oil, thymol, eucalyptus citriodora, camphor, carvacrol and combinations thereof.

5. Composition for a medical use according to any of the preceding claims, **characterized in that** the composition is in the form of a lotion, cream, ointment or gel.

6. Composition for a medical use according to any of the preceding claims, **characterized in that** the composition is absorbed into the nail within 200 seconds, preferably within 120 seconds, more preferably within 60 seconds, even more preferably within 45 seconds, most preferably within 30 seconds.

7. Composition for a medical use according to any of the preceding claims, **characterized in that** the composition is applied to the nail in an amount of more than 0,5 mg/cm2, preferably in an amount of more than 2,0 mg/cm2, more preferably in an amount of 4,5 mg/cm2.

8. Device comprising the composition for a medical use according to any of the preceding claims 1 to 5, wherein the device is adapted to apply the composition to the to be treated area by dripping or smearing.

## Patentansprüche

1. Zusammensetzung zur Verwendung als Medikament, wobei die Zusammensetzung auf einen Nagel aufgebracht wird, und wobei die Zusammensetzung aus folgendem besteht:
- einem wassermischbaren organischen Lösungsmittel, das Ethanol ist,
- Wasser,
- einem Terpen und/oder etherischem Öl,
- einem Zusatz, der zur Behandlung von Nagelerkrankungen geeignet ist, der Milchsäure ist,
- und gegebenenfalls Harnstoff als weiterem Zusatz, wobei:
- das wassermischbare organische Lösungsmittel in einer Menge von mehr als 50 Gew.% des Gesamtgewichts der Zusammensetzung vorhanden ist;
- Wasser in einer Menge von 10 Gew.% bis 25 Gew.% des Gesamtgewichts der Zusammensetzung vorhanden ist; und
- die Gesamtmenge an Terpen und etherischem Öl 1 Gew.% bis weniger als 15 Gew.% des Gesamtgewichts der Zusammensetzung beträgt.

2. Zusammensetzung zur Verwendung in der Behandlung von Onychomykose, wobei die Zusammensetzung auf einen Nagel aufgebracht wird, und wobei die Zusammensetzung besteht aus:
- einem wassermischbaren organischen Lösungsmittel, das Ethanol ist,
- Wasser,
- einem Terpen und/oder etherischem Öl,
- einem Zusatz, der zur Behandlung von Nagelerkrankungen geeignet ist, der Milchsäure ist,
- und gegebenenfalls Harnstoff als weiterem Zusatz, wobei:
- das wassermischbare organische Lösungsmittel in einer Menge von mehr als 50 Gew.% des Gesamtgewichts der Zusammensetzung vorhanden ist;
- Wasser in einer Menge von 10 Gew.% bis 25 Gew.% des Gesamtgewichts der Zusammensetzung vorhanden ist; und
- die Gesamtmenge an Terpen und etherischem Öl 1 Gew.% bis weniger als 15 Gew.% des Gesamtgewichts der Zusammensetzung beträgt.

3. Zusammensetzung für eine medizinische Verwendung nach einem der vorhergehenden Ansprüche, **dadurch gekennzeichnet, dass** Wasser in einer Menge von 10 Gew.% bis 20 Gew.% des Gesamtgewichts der Zusammensetzung vorhanden ist, wobei Wasser am meisten bevorzugt in einer Menge von 10 Gew.%, 15 Gew.% oder 20 Gew.% des Gesamtgewichts der Zusammensetzung vorhanden ist.

4. Zusammensetzung für eine medizinische Verwendung nach einem der vorhergehenden Ansprüche, **dadurch gekennzeichnet, dass** das Terpen und das etherische Öl ausgewählt sind aus Thymianöl, Thymol, Eucalyptus citriodora, Kampher, Carvacrol und Kombinationen davon.

5. Zusammensetzung für eine medizinische Verwendung nach einem der vorhergehenden Ansprüche, **dadurch gekennzeichnet, dass** die Zusammensetzung in Form einer Lotion, einer Creme, einer Salbe oder eines Gels vorliegt.

6. Zusammensetzung für eine medizinische Verwendung nach einem der vorhergehenden Ansprüche, **dadurch gekennzeichnet, dass** die Zusammensetzung innerhalb von 200 Sekunden, vorzugsweise innerhalb von 120 Sekunden, bevorzugter innerhalb von 60 Sekunden, noch bevorzugter innerhalb von 45 Sekunden, am meisten bevorzugt innerhalb von 30 Sekunden in den Nagel adsorbiert wird.

7. Zusammensetzung für eine medizinische Verwendung nach einem der vorhergehenden Ansprüche, **dadurch gekennzeichnet, dass** die Zusammensetzung in einer Menge von mehr als 0,5 mg/cm², vorzugsweise in einer Menge von mehr als 2,0 mg/cm², bevorzugter in einer Menge von 4,5 mg/cm², auf den Nagel aufgebracht wird.

8. Vorrichtung, umfassend die Zusammensetzung für eine medizinische Verwendung nach einem der vorhergehenden Ansprüche 1 bis 5, wobei die Vorrichtung adaptiert ist, um die Zusammensetzung durch Tropfen oder Schmieren auf den zu behandelnden Bereich aufzutragen.

## Revendications

1. Composition à utiliser comme médicament, la composition étant appliquée à un ongle, et la composition consistant en :
- un solvant organique miscible à l'eau qui est l'éthanol ;
- de l'eau ;
- un terpène et/ou une huile éthérique ;
- un additif approprié pour le traitement de maladies de l'ongle qui est l'acide lactique ;
- et éventuellement de l'urée comme additif supplémentaire,
dans laquelle :
- le solvant organique miscible à l'eau est présent dans une quantité de plus de 50 % en poids du poids total de la composition ;
- l'eau est présente dans une quantité de 10 % à 25 % en poids du poids total de la composition ; et
- la quantité totale de terpène et d'huile éthérique est de 1 % à moins de 15 % en poids du poids total de la composition.

2. Composition à utiliser dans le traitement de l'onychomycose, la composition étant appliquée à un ongle, et la composition consistant en :
- un solvant organique miscible à l'eau qui est l'éthanol ;
- de l'eau ;
- un terpène et/ou une huile éthérique ;
- un additif approprié pour le traitement de maladies de l'ongle qui est l'acide lactique ;
- et éventuellement de l'urée comme additif supplémentaire,
dans laquelle :
- le solvant organique miscible à l'eau est présent dans une quantité de plus de 50 % en poids du poids total de la composition ;
- l'eau est présente dans une quantité de 10 % à 25 % en poids du poids total de la composition ; et
- la quantité totale de terpène et d'huile éthérique est de 1 % à moins de 15 % en poids du poids total de la composition.

3. Composition pour un usage médical selon l'une quelconque des revendications précédentes, **caractérisée en ce que** l'eau est présente dans une quantité de 10 % à 20 % en poids du poids total de la composition, idéalement l'eau est présente dans une quantité de 10 %, 15 % ou 20 % en poids du poids total de la composition.

4. Composition pour un usage médical selon l'une quelconque des revendications précédentes, **caractérisée en ce que** le terpène et l'huile éthérique sont choisis parmi l'huile de thym, le thymol, l'eucalyptus citriodora, le camphre, le carvacrol et les combinaisons de ceux-ci.

5. Composition pour un usage médical selon l'une quelconque des revendications précédentes, **caractérisée en ce que** la composition se présente sous la forme d'une lotion, d'une crème, d'une pommade ou d'un gel.

6. Composition pour un usage médical selon l'une quelconque des revendications précédentes, **caractérisée en ce que** la composition est absorbée à l'intérieur de l'ongle dans les 200 secondes, de préférence dans les 120 secondes, mieux dans les 60 secondes, mieux encore dans les 45 secondes, idéalement dans les 30 secondes.

7. Composition pour un usage médical selon l'une quelconque des revendications précédentes, **caractérisée en ce que** la composition est appliquée à l'ongle dans une quantité de plus de 0,5 mg/cm², de préférence dans une quantité de plus de 2,0 mg/cm², mieux encore dans une quantité de 4,5 mg/cm².

8. Dispositif comprenant la composition pour un usage médical selon l'une quelconque des revendications 1 à 5 précédentes, le dispositif étant adapté pour appliquer la composition à la zone à traiter par application de gouttes ou badigeonnage.
